# EUROPEAN PATENT APPLICATION

(11) **EP 0 539 091 A1**
(43) Date of publication of application: **28.04.1993**
(21) Application number: 92309389.2
(22) Date of filing: 15.10.1992
(51) Int. Cl.: C07D 275/02, A01N 43/80

(54) **Novel bis-isothiazolones and the use thereof as microbicides**

(30) Priority: 24.10.1991 US 782032; 24.09.1992 US 949961
(71) Applicant: ROHM AND HAAS COMPANY, Philadelphia Pennsylvania 19105 (US)
(72) Inventor: Petigara, Ramesh Balubhai, Hatfield, Pennsylvania 19440 (US); Lange, Barry Clifford, Lansdale, Pennsylvania 19446 (US); Osei-Gyimah, Horsham, Pennsylvania 19044 (US)
(74) Representative: Smith, Julian Philip Howard

(57) **Abstract**

Compounds of the formula I 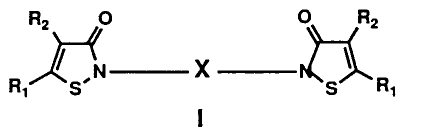
where
R₁ is hydrogen or halogen;
R₂ is hydrogen or halogen;
X is selected from the group consisting of
(i) (C₂-C₂₀)alkyl, optionally substituted with one or more (C₁-C₄) alkyl groups, optionally containing oxygen heteroatoms or carbonyl groups, or or (C₂-C₂₀) straight chain alkylene, optionally substituted with one or more (C₁-C₄) alkyl groups; or
(ii)

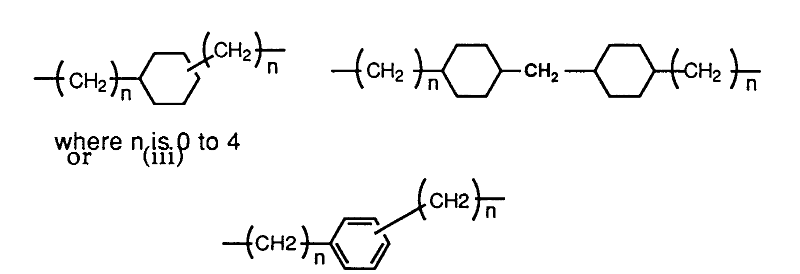

where n is 0 to 4, and the aromatic group is optionally substituted by one or more (C₁-C₄)alkyl, nitro, halo, cyano, or methoxy groups are prepared by simultaneous cyclization of the two mercaptopropionamide groups of a bis-amide of the formula (NHCOCH₂CH₂SH)₂ .

## Description

This invention concerns antimicrobial compounds and compositions. In particular, it relates to improvements in the field of isothiazolone compounds which are useful as microbicides.

Lewis et al., U.S.-A-3,761,488, assigned to Rohm and Haas Co., the same assignee as the present invention, discloses 3-isothiazolone compounds, among which is compound number 68, 2-[β-(N-isothiazolonyl)ethyl]-3-isothiazolone, also called ethylidene-bis-3-isothiazolone which has a structure similar to formula I below except X would be 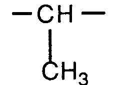
Column 5, line 58 to Column 6, line 2 discloses a method of making compound 68 by reacting vinyl acetate with a 3-hydroxyisothiazole to make a 2-vinyl-3-isothiazolone which is then reacted with additional 3-hydroxyisothiazole to form ethylidene-bis-3-isothiazolone. This reaction would not be suitable to make bis-3-isothiazolones other than the ethylidene.

Lewis et al., US-A-3,544,580, also assigned to Rohm and Haas Company, discloses that 3-isothiazolones react with diacyl halides such as adipoyl chloride to give a mixture of N,N' and O,O'-bis compounds, bis-(3-isothiazolyl)adipate and adipoyl-bis-3-isothiazolone:

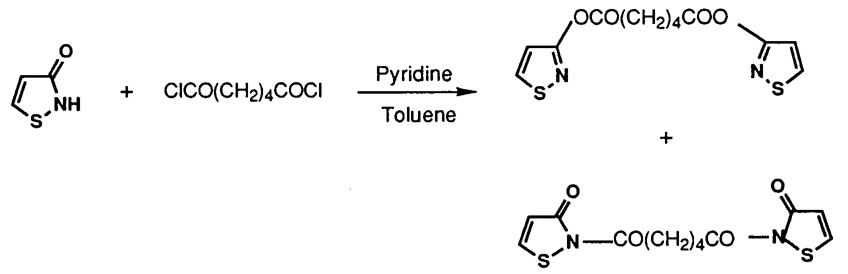

Other related Lewis et al patents are U.S.-A-3,835,150; 3,706,757; and 4,105,431.

Many known microbicides have toxicity and /or environmental problems, and one object of the present invention is to provide novel compounds which may overcome some of these problems. Thus in one aspect the present invention provides compounds of the formula **I** 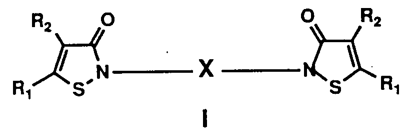
where
R₁ is hydrogen or halogen;
R₂ is hydrogen or halogen;
X is
(i) (C₂-C₂₀)alkyl, optionally substituted with one or more (C₁-C₄) alkyl groups which optionally contain oxygen heteroatoms or carbonyl groups, or (C₂-C₂₀) straight chain alkylene, optionally substituted with one or more (C₁-C₄) alkyl groups;
or (ii) 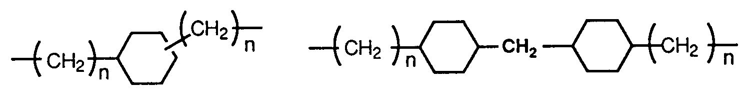 where n is 0 to 4
or (iii) 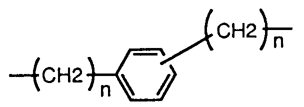 where n is 0 to 4, and the aromatic group is optionally substituted by one or more (C₁-C₄)alkyl, nitro, halo, cyano or methoxy groups.

In one preferred embodiment X is (C₈-C₂₀) straight chain alkylene, optionally substituted with one or more (C₁-C₄) alkyl groups.

In another aspect the invention comprises a process of preparing a compound of formula I comprising simultaneous cyclization of the two mercaptopropionamide groups of a bis-amide of the formula X₁ (NHCOCH₂CH₂SH)₂.

The compounds of the invention are especially active and efficient antimicrobial compounds. Among the preferred compounds of formula I are the following:

**TABLE 1**

| Comp'd No. | R₁ | R₂ | X | Melting Point (°C) |
|---|---|---|---|---|
| 1 | Cl | H | (CH₂)₂ | 182-185 |
| 2 | Cl | Cl | (CH₂)₂ | 201-203 |
| 3 | Cl | H | (CH₂)₄ | 122-124 |
| 4 | Cl | Cl | (CH₂)₄ | 169-171 |
| 5 | Cl | H | (CH₂)₆ | 127-130 |
| 6 | Cl | Cl | (CH₂)₆ | 123-125 |
| 7 | Cl | H | (CH₂)₈ | 91-93 |
| 8 | Cl | Cl | (CH₂)₈ | 117-120 |
| 9 | Cl | Cl | (CH₂)₉ | 104-106 |
| 10 | Cl | H | (CH₂)₁₀ | 91-93 |
| 11 | Cl | Cl | (CH₂)₁₀ | 126-127 |
| 12 | Cl | H | (CH₂)₁₂ | 95-97 |
| 13 | Cl | Cl | (CH₂)₁₂ | 114-117 |
| 14 | Cl | H | 1,4-Xylyl | 146-149 |
| 15 | Cl | H | 1,4-Dimethylenecyclohexane | 186-189 |

The compounds of Formula **I** may be prepared by the simultaneous cyclization of the two mercaptopropionamide groups of the bis-amide of Formula **II**

X₁(NHCOCH₂CH₂SH)₂ **II**

where **X₁** is any of the groups embraced by **X** in Formula **I** as defined above.

The bis-amide of Formula **II** can be prepared, for example, by the method described by E.R. Atkinson, et. al., j. Med. Chem., 8, 29 (1965), which consists of reacting a diamine with methyl 3-mercaptopropionate in methanol. The reaction mixture is held at 40-50°C for 1-4 days after which it is cooled to precipitate the bis-amide. The solid is removed by filtration and washed with ether. Physical data of representative compounds of formula **II** are given below in Table 2.

| TABLE 2 - Structure and Physical Data of Representative Compounds of Formula II | | |
|---|---|---|
| Compound No. | X | Melting Point |
| 16 | (CH₂)₂ | 153-156 °C |
| 17 | (CH₂)₄ | 156-160 °C |
| 18 | (CH₂)₆ | 144-146 °C |
| 19 | (CH₂)₈ | 128-131 °C |
| 20 | (CH₂)₉ | 120-123 °C |
| 21 | (CH₂)₁₀ | 116-120 °C |
| 22 | (CH₂)₁₂ | 102-106 °C |
| 23 | 1,4-Xylyl | 162-166 °C |
| 24 | 1,4-Dimethylenecyclohexane | 155-158 °C |

The cyclization of the mercaptopropionamide moieties of formula II occurs simultaneously to give the bis-isothiazolones of formula I. The cyclization is accomplished by reacting the bis-amide with a halogenating agent according to the method described by S. N. Lewis , et al., US 3,761,488. Typical halogenating agents include chlorine, bromine, sulfuryl bromide and sulfuryl chloride. Chlorine and sulfuryl chloride are the preferred halogenating agents. The bis-amide is treated with 3-10 equivalents of the halogenating agent to form the isothiazolone rings. In the process, a halogen atom is introduced at the 5, or at the 4 and 5 positions of the isothiazolone rings. The cyclization takes place within 1-24 hours, at a broad temperature range of -10-100 °C. A preferred temperature range is 0-50 °C.

The reaction is carried out in an inert solvent such as toluene, xylene, ethylene dichloride or ethyl acetate. Ethyl acetate is the preferred solvent.

The compounds of the invention can be used in combination with other microbicides. The term "microbicide" is considered equivalent to "antimicrobial" as used herein. Suitable methods of application of the compounds of the invention to control fungi, bacteria, algae, viruses, yeasts, and the like are in amounts and with carriers, etc., which are well known in the art.

The following examples are presented to illustrate a few embodiments of the invention, but are not to be considered as limiting.

### Example 1

### N,N'-Octamethylene-bis-(3-mercaptopropionamide) (Compound 19)

Methyl-3-mercaptopropionate (100 g., 0.83 mole) was added in one portion to a stirred solution of 1,8-diaminooctane (36.0 g., 0.25 mole) in 100 ml of dry methanol. The solution was heated to 40°C and held there fore 60 hours and then cooled to 0°C. A white solid precipitate formed and the mixture was diluted with ether. The solid was removed by filtration and washed several times by trituration in ether. The bis-amide was obtained as white solid; 46.2 g.; mp 128-131°C; IR (KBr) 3300, 1630 cm⁻¹.

### Example 2

### 1,8-Bis-(5-Chloro-4-Isothiazolin-3-one-2-yl)Octane (Compound 7)

To dry ethyl acetate (200 ml), magnetically stirred at 0°C, was added concurrently, the bis-amide of Example 1 above (20.0 g., 0.062 mole) and sulfuryl chloride (25 ml, 0.31 mole), each in 24 portions over 1 hour period. The mixture was kept at 0°C during the additions and subsequently for 1 additional hour and then allowed to warm to room temperature. After stirring for 3 hours, the mixture was filtered. The gummy solid was extracted thoroughly with chloroform. The chloroform solution was washed three times with water, dried, and concentrated. The residual oil was purified by column chromatography on silica-gel, using EtOAc/CHCl3 (1/1) as eluent. Compound 7 was obtained as white solid, 3.2 g; mp 91-93°C; IR(KBr) 1635 cm¹; NMR(CDCl3) d 6.25 (s, 2H); 3.8 (t, 4H); 1.65 (m, 4H); 1.35 (br, s, 8H).

### Example 3

### 1,4-Bis-(4,5-Dichloro-4-isothiazolin-3-one-2-yl)butane (Compound 4)

To dry ethyl acetate (200 ml), magnetically stirred at room temperature, was added concurrently the bis-amide, 17, (20.0 g., 0.076 mole) and sulfuryl chloride (30.5 ml, 0.38 mole), each in 24 equal portions. The reaction temperature which rose to 35°C during the addition was increased to 40°C and held there for 3 hours. Upon cooling, the mixture was filtered, and the solid extracted thoroughly with chloroform. The chloroform extract was washed with water and dried (MgSO₄). The filtrate was similarly washed with water and dried. The combined organic solution was concentrated. The solid residue was suspended in methanol / ether solution, cooled for several hours and removed by filtration. The solid was purified by column chromatography on silica-gel using EtOAc/CHCl₃ (1/1) as eluent. Compound 4 was obtained as a white solid; 5.4 g; mp 169-171°C; IR(KBr) 1630 cm⁻¹; NMR (CDCl₃) d 3.9 (m, 4H); 1.75 (m, 4H).

### Example 4

### Biocidal Activity

Efficacy against bacteria and fungi was studied. A minimum inhibitory concentration (MIC) value was obtained using Trypticase Soy Broth, pH 7.0 and preparing a serial dilution with a starting concentration of 500 ppm. A stock solution of the test compound was made in dimethyl sulfoxide/acetone/water mixture. The test organisms used to demonstrate biocidal activity are listed in Table 3. The MICs of the compounds of this invention against the test organisms are shown in Table 4.

| Table 3: Microorganisms used in the Antimicrobial Test | |
|---|---|
| Name | Abbreviations Used |
| **Bacteria** | |
| Pseudomonas aeruginosa | Psae |
| Escherichia coli | Ecol |
| Staphlococcus aureus | Saur |
| **Fungi** | |
| Aspergillus niger | Anig |

| TABLE 4: Antimicrobial Activity (MIC) of Compounds of Formula I | | | | |
|---|---|---|---|---|
| Comp.No. | Psae | Ecol | Saur | Anig |
| 1 | 125 | 8 | 63 | <0.5 |
| 2 | >250 | 32 | 2 | 250 |
| 3 | 63 | 8 | 63 | 4 |
| 4 | 125 | 16 | 63 | 16 |
| 5 | 250 | 8 | 32 | 1 |
| 5 | >250 | 16 | 16 | 4 |
| 7 | 32 | 4 | 16 | 1 |
| 8 | 250 | 63 | 4 | 2 |
| 9 | >500 | >500 | <0.25 | 500 |
| 10 | 8 | 8 | <0.25 | 4 |
| 11 | 125 | 63 | 4 | 63 |
| 12 | 32 | 500 | <0.25 | 16 |
| 13 | 16 | 32 | 0.5 | 32 |
| 14 | 8 | 8 | <0.25 | 16 |
| 15 | 500 | 63 | 2 | 63 |

## Claims

1. Compound of the formula **I** 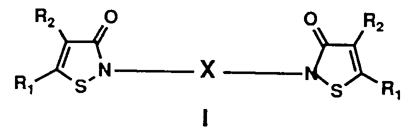 where
R₁ is hydrogen or halogen;
R₂ is hydrogen or halogen;
X is
(i) (C₂-C₂₀)alkyl, optionally substituted with one or more (C₁-C₄) alkyl groups which optionally contain oxygen heteroatoms or carbonyl groups, or (C₂-C₂₀) straight chain alkylene, optionally substituted with one or more (C₁-C₄) alkyl groups;
or (ii) 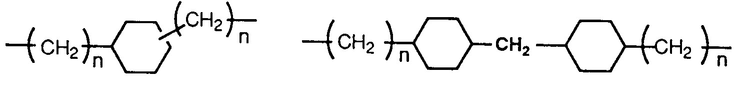 where n is 0 to 4
or (iii) 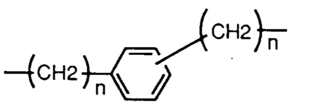 where n is 0 to 4, and the aromatic group is optionally substituted by one or more (C₁-C₄)alkyl, nitro, halo, cyano or methoxy groups.

2. Compound according to claim 1 wherein X is (C₈-C₂₀) straight chain alkylene, optionally substituted with one or more (C₁-C₄) alkyl groups.

3. Compound according to claim 1 which is 1,2-Bis-(5-chloro-4-isothiazolin-3-one-2-yl)ethane;
1,2-Bis-(4,5-dichloro-4-isothiazolin-3-one-2-yl)ethane;
1,4-Bis-(5-chloro-4-isothiazolin-3-one-2-yl)butane;
1,4-Bis-(4,5-dichloro-4-isothiazolin-3-one-2-yl)butane;
1,6-Bis-(5-chloro-4-isothiazolin-3-one-2-yl)hexane;
1,6-Bis-(4,5-dichloro-4-isothiazolin-3-one-2-yl)hexane;
1,8-Bis-(5-chloro-4-isothiazolin-3-one-2-yl)octane;
1,8-Bis-(4,5-dichloro-4-isothiazolin-3-one-2-yl)octane;
1,9-Bis-(4,5-dichloro-4-isothiazolin-3-one-2-yl)nonane;
1,10-Bis-(5-chloro-4-isothiazolin-3-one-2-yl)decane;
1,10-Bis-(4,5-dichloro-4-isothiazolin-3-one-2-yl)decane;
1,12-Bis-(5-chloro-4-isothazolin-3-one-2-yl)dodecane;
1,12-Bis-(4,5-dichloro-4-isothiazolin-3-one-2-yl)dodecane;
1,4-Bis-(5-chloro-4-isothiazolin-3-one-2-yl)xylene; or
1,4-Bis-(5-chloro-4-isothiazolin-3-one-2-yl-methylene) cyclohexane.

4. Composition comprising a compound as defined in any preceding claim and an agronomically or pharmaceutically acceptable carrier.

5. Method for preventing or inhibiting the growth of bacteria, fungi, algae, yeast or viruses in a locus susceptible or subject to contamination thereby, comprising incorporating into or onto the locus, in an amount effective to adversely affect said growth, a compound or composition as defined in any preceding claim.

6. Use of a compound or compositionas defined in any of claims 1 to 4 as a microbicide.

7. Process for preparing a compound as defined in claims 1, 2 or 3 comprising simultaneously cyclizing the two mercaptopropionamide groups of a bis-amide of the formula X₁(NHCOCH₂CH₂SH)₂.

8. Process according to claim 7 wherein said cyclization is conducted by reacting between 4 and 8 equivalents of Cl₂ or SO₂Cl₂ with said bis-amide.

9. Process according to claim 8 wherein about 4 equivalents of chlorinating agent are used and R₁ and R₂ are each H, or wherein about 6 equivalents of chlorinating agent are used and R₁ is C₁ and R₂ is H, or wherein about 8 equivalents of chlorinating agent are used and R₁ and R₂ are both Cl.
